# EUROPEAN PATENT APPLICATION

(11) **EP 4 834 279 A1**
(43) Date of publication of application: **04.11.2026**
(21) Application number: 24910887.9
(22) Date of filing: 18.12.2024
(51) Int. Cl.: A61F 2/07

(54) **COVERED STENT AND DELIVERY SYSTEM**

(30) Priority: 27.12.2023 CN 202311838041
(71) Applicant: Lifetech Scientific (Shenzhen) Co., Ltd., Shenzhen, Guangdong 518063 (CN)
(72) Inventor: XU, Yang, Shenzhen, Guangdong 518063 (CN); TANG, Jiangfeng, Shenzhen, Guangdong 518063 (CN)
(74) Representative: Prinz & Partner mbB
(86) International application number: PCT/CN2024/140284
(87) International publication number: WO 2025/139956

(57) **Abstract**

Provided are a covered stent and a delivery system. The covered stent includes a main stent having a tubular body and an embedded stent. The main stent includes a proximal section, an aneurysm sac section, and a distal section in an axial direction. The aneurysm sac section includes a first channel and a second channel which are arranged in a radial direction, the second channel is in communication with the distal section, and the first channel is provided with an opening at a distal end and is internally provided with the embedded stent. By incorporating the embedded stent, an implantation channel for an internal iliac artery stent is provided, thereby offering enhanced anchorage for the internal iliac artery stent. The total support strength of the first channel and the embedded stent is lower than the support strength of the second channel, and since the second channel has the higher support strength, the first channel deforms before the second channel when the covered stent is compressed, thereby allowing the second channel to maintain a more stable form, preventing compression-induced occlusion, and ensuring smooth passage of blood through the second channel.

## Description

### Technical Field

The present invention relates to the technical field of medical devices, and in particular, to a covered stent and a delivery system.

### Background Art

The iliac artery includes the common iliac artery, external iliac artery, and internal iliac artery. In the current treatment of iliac artery aneurysms, endovascular therapy can be employed to implant an iliac artery bifurcation stent and an internal iliac covered stent to reconstruct arterial blood vessels. The iliac artery bifurcation stent in the prior art typically has two branch channels which are respectively configured to reconstruct the internal iliac artery and the external iliac artery. To ensure that the internal iliac artery channel is not occluded due to vascular compression, the wave-like design of the internal iliac artery channel provides certain support strength. In practice, the patency of the external iliac artery channel is far more critical than that of the internal iliac artery channel. Therefore, a larger lumen is typically designed for the external iliac artery to ensure optimal blood flow. However, an excessively large lumen of the external iliac artery channel places higher demands on the patient's vascular anatomy, which is not conductive to expansion of the scope of application of the device.

### Summary of the Invention

Based on this, it is necessary to provide a new covered stent, which can provide an implantation channel for an internal iliac stent, and also can enable an external iliac channel to have better support strength when the stent is compressed, to maintain an entire form of the external iliac channel.

A covered stent includes a main stent having a tubular body and an embedded stent, and the main stent includes a proximal section, an aneurysm sac section, and a distal section in an axial direction; the proximal section is in communication with the distal section through the aneurysm sac section; the aneurysm sac section includes a first channel and a second channel which are arranged in a radial direction, the second channel is in communication with the distal section, and a distal end of the first channel is provided with an opening in communication with the outside; the embedded stent is arranged in the first channel, the embedded stent is at least partially connected to a side wall of the first channel, and a distal end of the embedded stent is in communication with the opening; and the total support strength of the first channel and the embedded stent is lower than the support strength of the second channel.

In one embodiment, the aneurysm sac section includes a plurality of first corrugated rings arranged at intervals in the axial direction, a number of waves of the plurality of first corrugated rings is the same, and crests and/or troughs of adjacent first corrugated rings are arranged opposite to each other.

In one embodiment, the first corrugated ring has an opening at least in a portion corresponding to the first channel.

In one embodiment, a wave angle of the first corrugated ring in a portion corresponding to the second channel is greater than a wave angle of the first corrugated ring in a portion corresponding to the first channel, or a wire diameter of the first corrugated ring in the portion corresponding to the second channel is larger than a wire diameter of the first corrugated ring in the portion corresponding to the first channel.

In one embodiment, the first corrugated ring includes a plurality of first wave struts, and a support member is arranged between adjacent first wave struts, and a plurality of support members are configured such that a compressible distance between the first wave struts located in a portion corresponding to the second channel is smaller than a compressible distance between the first wave struts located in a portion corresponding to the first channel.

In one embodiment, a gap is formed between the support member and the adjacent first wave strut, and the gap in the portion corresponding to the second channel is smaller than the gap in the portion corresponding to the first channel.

In one embodiment, the support member is an elastic support member, two sides of the elastic support member are respectively connected to two adjacent first wave struts, and an elastic modulus of the elastic support member at the second channel is greater than an elastic modulus of the elastic support member at the first channel.

In one embodiment, the proximal section includes a plurality of proximal support corrugated rings arranged at intervals in the axial direction, the distal section includes a plurality of distal support corrugated rings arranged at intervals in the axial direction, and the embedded stent includes a mesh main body.

In one embodiment, a first covering membrane covers a surface of the main stent, a second covering membrane covers a surface of the embedded stent, and the support strength of the first covering membrane is higher than the support strength of the second covering membrane.

In one embodiment, the support strength of the aneurysm sac section located at a proximal end and a distal end is higher than the support strength of the aneurysm sac section at a middle position.

In one embodiment, the support strength of the aneurysm sac section located at a proximal end or a distal end is higher than the support strength of the aneurysm sac section at a middle position.

The present invention has the following beneficial effects. Compared with the prior art, the present invention provides the covered stent, the covered stent includes the main stent having the tubular body and the embedded stent, and the main stent includes the proximal section, the aneurysm sac section, and the distal section in the axial direction; the aneurysm sac section includes the first channel and the second channel which are arranged in the radial direction, the second channel is in communication with the distal section, and the first channel is provided with the opening at a distal end and is internally provided with the embedded stent; by incorporating the embedded stent, an implantation channel for an internal iliac artery stent is provided, thereby offering enhanced anchorage for the internal iliac artery stent. The total support strength of the first channel and the embedded stent is lower than the support strength of the second channel, and since the second channel has the higher support strength, the first channel deforms before the second channel when the covered stent is compressed, thereby allowing the second channel to maintain a more stable form, preventing compression-induced occlusion, and ensuring smooth passage of blood through the second channel.

### Brief Description of the Drawings

FIG. 1 is a schematic diagram of an overall structure of a covered stent according to Embodiment 1 of the present invention;
FIG. 2 is a schematic structural diagram of a main stent according to Embodiment 1 of the present invention;
FIG. 3 is a schematic structural diagram of an embedded stent according to Embodiment 1 of the present invention;
FIG. 4 is a schematic structural diagram of distribution of wave angles of a first corrugated ring according to Embodiment 1 of the present invention;
FIG. 5 is a schematic structural diagram of a first corrugated ring with gradually reduced wave angles according to Embodiment 1 of the present invention;
FIG. 6 is a schematic structural diagram of wire diameter distribution of a first corrugated ring according to Embodiment 1 of the present invention;
FIG. 7 is a schematic structural diagram of support members with different lengths of a first corrugated ring according to Embodiment 2 of the present invention;
FIG. 8 is a schematic structural diagram of support members with different heights of a first corrugated ring according to Embodiment 2 of the present invention;
FIG. 9 is a schematic diagram of a structure in which a support member is an elastic support member according to Embodiment 2 of the present invention;
FIG. 10 is a schematic structural diagram of covered stents in other embodiments according to Embodiment 2 of the present invention;
FIG. 11 is a schematic structural diagram of a main stent according to Embodiment 3 of the present invention;
FIG. 12 is a schematic structural diagram of a first corrugated ring provided with an opening according to Embodiment 3 of the present invention;
FIG. 13 is a schematic diagram of an overall structure of a covered stent according to Embodiment 4 of the present invention;
FIG. 14 is a schematic structural diagram of a first embedded stent and a second embedded stent having different wire diameters according to Embodiment 4 of the present invention;
FIG. 15 is a partially enlarged view of position A in FIG. 14 according to the present invention;
FIG. 16 is a schematic structural diagram of a first embedded stent and a second embedded stent having different grid densities according to Embodiment 5 of the present invention;
FIG. 17 is a schematic diagram of a first channel and a second channel of an aneurysm sac section having different axial lengths according to Embodiment 6 of the present invention;
FIG. 18 is a schematic structural diagram of a flat opening of an exposed section of a second embedded stent according to Embodiment 6 of the present invention;
FIG. 19 is a schematic structural diagram of an oblique opening of an exposed section of a second embedded stent according to Embodiment 6 of the present invention;
FIG. 20 is a schematic structural diagram of a variable-diameter proximal corrugated ring arranged at a proximal end of an aneurysm sac section according to Embodiment 6 of the present invention;
FIG. 21 is a schematic structural diagram of other covered stents according to Embodiment 6 of the present invention;
FIG. 22 is a schematic structural diagram of a covered stent according to Embodiment 7 of the present invention;
FIG. 23 is a schematic structural diagram of a main stent according to Embodiment 7 of the present invention;
FIG. 24 is a schematic diagram of a first embedded stent and a second embedded stent having different axial lengths according to Embodiment 7 of the present invention;
FIG. 25 is a schematic structural diagram of a triangular corrugated ring stent according to Embodiment 7 of the present invention;
FIG. 26 is a schematic structural diagram of developing members arranged at two ends of a first embedded stent and a second embedded stent according to Embodiment 7 of the present invention;
FIG. 27 is a schematic diagram of a structure in which a transition stent adopts a corrugated ring stent according to Embodiment 7 of the present invention;
FIG. 28 is a schematic diagram of a structure in which a transition stent adopts a mesh braided stent according to Embodiment 7 of the present invention;
FIG. 29 is a schematic structural diagram of a special-shaped corrugated ring according to Embodiment 8 of the present invention;
FIG. 30 is a schematic structural diagram of gradually reduced distal high waves of a special-shaped corrugated ring according to Embodiment 8 of the present invention;
FIG. 31 is a schematic structural diagram of a hook member and a hook rod according to Embodiment 9 and Embodiment 10 of the present invention;
FIG. 32 is a schematic structural diagram of a stent delivery system according to Embodiment 10 of the present invention; and
FIG. 33 is a schematic structural diagram of a covered stent arranged in a delivery device according to Embodiment 10 of the present invention.

### Detailed Description of the Invention

To better understand the concept of the present invention, implementations of the present invention are described in detail below with reference to the drawings, and the following specific embodiments are only part of the embodiments of the present invention, but not limitations to the present invention.

For ease of description, spatial relative relationship terms may be used in the text to describe a relationship between one element or feature and another element or feature as shown in the figure. These relative relationship terms include, for example, "interior", "exterior", "inner side", "outer side", "below", "under", "above", "over", and the like. Such spatially relative relationship terms are meant to include different orientations of a device in use or operation other than orientations depicted in the figures. For example, if the device in the figures is overturned, an element described as "under" or "below" other elements or features will then be oriented as "above" or "over" other elements or features. Accordingly, the example term "below" may include both an upper orientation and a lower orientation. The device may otherwise be oriented (rotated 90 degrees or in other directions) and a spatial relative relationship descriptor used herein is interpreted accordingly.

Although terms such as first, second, and third may be used herein to describe a plurality of elements, components, areas, layers, and/or sections, these elements, components, areas, layers, and/or sections should not be limited by these terms. These terms may only be configured to distinguish one element, component, area, layer, or section from another element, component, area, layer, or section. The terms such as "first", "second", and other numerical terms, when used herein, do not imply an order or a sequence unless otherwise specified in the context. Therefore, a first element, component, area, layer, or section discussed below may be referred to as a second element, component, area, layer, or section without departing from the teachings of example implementations.

To describe the structure of the present invention more clearly, terms "proximal end" and "distal end" are defined herein as terms commonly used in the field of interventional medicine. Specifically, the "distal end" indicates one end of a blood vessel away from the heart, and the "proximal end" indicates one end of a blood vessel close to the heart; "axial direction" indicates a length direction, and "radial direction" indicates a direction perpendicular to the "axial direction"; and "upper end" and "lower end" are two ends relatively away from each other, and when one end is defined as the "upper end", the other end away from this end is the "lower end".

### Embodiment 1:

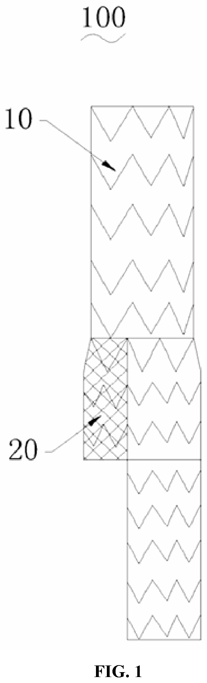

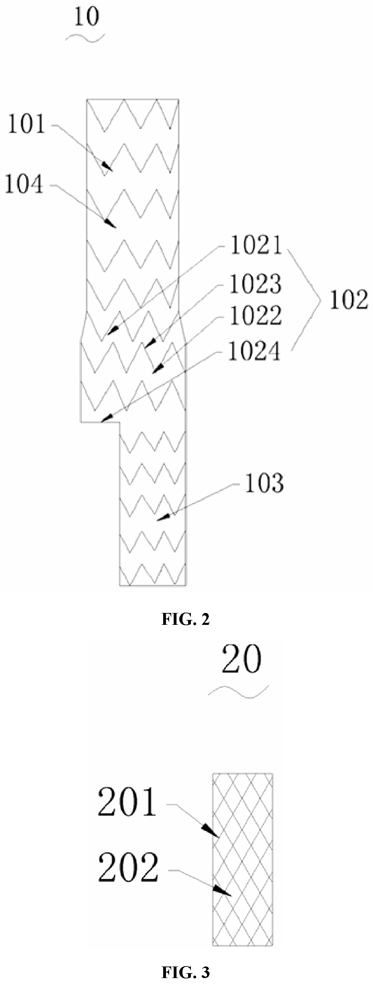

Referring to FIG. 1 to FIG. 2, the present invention provides a covered stent 100. The covered stent 100 usually includes a metallic framework and a covering membrane material. The metallic framework can adopt a Z-shaped wave or a braided mesh design, and the covering membrane material has a certain blood flow isolation ability, and is combined with the metallic framework in a pressurized heating or suturing manner to form a complete covered stent 100. A proximal end of the covered stent 100 is typically placed in a common iliac artery or connected to an abdominal aortic stent, and a lumen diameter is generally matched with a diameter of the common iliac artery. In this embodiment, referring to FIG. 1 and FIG. 3, the covered stent 100 includes a main stent 10 having a tubular body and an embedded stent 20, the main stent 10 is directly placed in a blood vessel to be in contact with a wall of the blood vessel, the embedded stent 20 is arranged in a lumen of the main stent 10 to shunt blood flow, a first covering membrane 104 covers a surface of the main stent 10, and a second covering membrane 202 covers a surface of the embedded stent 20, wherein the main stent 10 includes a proximal section 101, an aneurysm sac section 102, and a distal section 103 in an axial direction; and the proximal section 101 is connected to a proximal side of the aneurysm sac section 102, the distal section 103 is connected to the distal side of the aneurysm sac section 102, and the proximal section 101 is in communication with the distal section 103 through the aneurysm sac section 102. When the covered stent 100 is mounted in a diseased blood vessel, the aneurysm sac section 102 is generally placed at an aneurysm sac position of the blood vessel, the aneurysm sac section 102 is formed jointly by a second channel 1022 and a first channel 1021 which are arranged in a radial direction, and a radial width of the aneurysm sac section 102 is usually configured to be greater than a diameter of the distal section 103 and a diameter of the proximal section 101, such that the first channel 1021 and the second channel 1022 in the aneurysm sac section 102 both have sufficient circulation space to avoid complete occlusion after implantation due to compression by an aneurysm sac. The second channel 1022 and the first channel 1021 are isolated to form two relatively independent blood flow channels which are respectively an external iliac channel and an internal iliac channel. During use, the first channel 1021 is configured to allow an internal iliac stent to deploy, and the second channel 1022 is configured to connect the extended distal section 103 to establish fluid communication with an external iliac artery. In this embodiment, by arranging the embedded stent 20 in the first channel 1021, the first channel 1021 and the second channel 1022 are relatively independently isolated. In addition, the distal end of the first channel 1021 is provided with an opening 1024 in communication with the outside, and a distal end of the embedded stent 20 is in communication with the opening 1024; and the arrangement of the embedded stent 20 can allow a form of the formed first channel 1021 to match a form of the internal iliac stent to be implanted, thereby ensuring that the stent has better adaptability during and after implantation. When the embedded stent 20 is arranged in the first channel 1021, the embedded stent 20 is at least partially connected to a side wall of the first channel 1021 of the aneurysm sac section 102. Preferably, the embedded stent 20 is connected to the aneurysm sac section 102 through multi-point adhesive bonding or suturing to form a surface-contact connection. Such configuration can ensure that at least the portion, connected to the aneurysm sac section 102, of the embedded stent 20 always maintains attached to the aneurysm sac section 102 of the main stent 10, and when the aneurysm sac section 102 is compressed, the attachment portion can deform together with deformation of the aneurysm sac section 102, thereby effectively preventing the embedded stent 20 from remaining undeformed and merely shifting in a lumen of the aneurysm sac section 102 when the aneurysm sac section 102 deforms, which may affect a width and patency of the second channel 1022.

In other embodiments, the covered stent 100 provided in the present invention is not only applied to reconstruct a common iliac artery, an internal iliac artery, and an external iliac artery, but also applied to reconstruct an abdominal aorta and a right subclavian artery in an aortic arch. When the covered stent is applied to reconstruct the abdominal aorta, if a lesion site of vascular diseases such as aortic aneurysms or dissections is located in an abdominal aortic region, the proximal section 101 of the covered stent 100 may be anchored to an inferior margin of a renal artery, the distal section 103 is anchored in the common iliac artery, the second channel 1022 is connected to the abdominal aorta and the common iliac artery, and by placing a stent in the first channel, the first channel 1021 is connected to the abdominal aorta and the common iliac artery. When the covered stent is applied to reconstruct the right subclavian artery, if lesion sites of the vascular diseases such as aortic aneurysms or dissections are located in right subclavian and right common carotid artery regions, the proximal section 101 of the covered stent 100 may be anchored to an innominate artery, the distal section 103 is anchored to a right common carotid artery, the second channel 1022 is connected to the right common carotid artery and the innominate artery, and by implanting a stent in the first channel, the first channel 1021 is connected to the right subclavian artery and the innominate artery. Alternatively, the proximal section 101 and the distal section 103 of the covered stent 100 provided by the present invention may not be directly anchored to blood vessels, but indirectly fixed to the blood vessels by anchoring to other stents. The above is merely example common application locations of the covered stent 100 provided in the present invention in the human body, and does not limit the application of the covered stent 100 provided in the present invention. When a type of a blood vessel and a lesion site are appropriate, the covered stent 100 provided in the present invention may also be applied to other bifurcated vascular locations in the human body.

In this embodiment, referring to FIG. 1 and FIG. 2, in order to ensure that when the aneurysm sac section 102 of the covered stent 100 is compressed, the first channel 1021 obtains certain support through the embedded stent 20, and at the same time, the second channel 1022 can maintain a good form to avoid occlusion due to excessive compression, the total support strength of the first channel 1021 and the embedded stent 20 of the aneurysm sac section 102 of the covered stent 100 in the present invention is configured to be lower than the support strength of the second channel 1022. In this way, under compressive force, a portion with lower support strength deforms first and has a large deformation amount, while a portion with higher support strength slowly deforms and has a limited deformation amount. Accordingly, the second channel 1022 has higher support strength and can ensure to have a smaller deformation amount than the first channel 1021 when subjected to the same compressive force, thereby maintaining the form of the second channel 1022; and the first channel 1021 deforms first and has a larger deformation amount due to its lower support strength, such that the deformation of the aneurysm sac section 102 mostly concentrates on the first channel 1021, and the form of the second channel 1022 is maintained. However, the embedded stent 20 is arranged in the first channel 1021, portions of the embedded stent 20, which are not connected to the aneurysm sac section 102 in the lumen, do not deform substantially, and a good channel form can still be maintained.

In this embodiment, referring to FIG. 3, the embedded stent 20 has a mesh main body 201 and a second covering membrane 202, wherein the mesh main body 201 adopts a mesh braided stent structure, and the mesh support structure can provide good tension, thereby maintaining a good form. In addition, when the internal iliac stent is implanted, the mesh main body 201 can provide more contact points, such that greater frictional force is formed between the internal iliac stent and the embedded stent 20, thereby effectively enhancing attachment force and anti-migration performance of the stent. Referring to FIG. 2, the proximal section 101 is provided with a plurality of proximal support corrugated rings 1011 in the axial direction, and the distal section 103 is provided with a plurality of distal support corrugated rings 1031 in the axial direction. The proximal support corrugated rings 1011 and the distal corrugated rings 1031 are all Z-shaped annular corrugated rings to provide supporting force. While the Z-shaped annular corrugated rings can provide the supporting force, covering membrane gaps are reserved between the corrugated rings, such that the stent can deform at the covering membrane gaps and has good flexibility. A diameter of each proximal support corrugated ring 1011 is greater than a diameter of each distal support corrugated ring 1031 to adapt to different diameters of blood vessels. In other embodiments, the mesh main body may also be a cutting mesh main body formed by cutting a tubular metal, such as a nickel-titanium tube and a stainless steel tube.

The support strength of the first covering membrane 104 is configured to be higher than the support strength of the second covering membrane 202. In this embodiment, the first covering membrane 104 is a polyethylene terephthalate (PET) membrane, and the second covering membrane 202 is an expanded polytetrafluoroethylene (ePTFE) membrane. The PET membrane has the characteristic of high tensile strength, while the ePTFE membrane has weak tensile strength and smooth surface, is not easy to form thrombus, is good for long-term patency of small blood vessels, and has small pores. The PET membrane and the ePTFE membrane are combined for use, the overall strength of the covering membranes of the stent is ensured, and the covered stent 100 is enabled to have a better effect of isolating blood flow, to ensure the long-term patency of branches and a good sealing effect. When the support strength of the first covering membrane 104 is higher than the support strength of the second covering membrane 202, such that the support strength of the covering membrane of the second channel 1022 is higher than the support strength of the embedded stent 20 of the first channel 1021, and the embedded stent 20 is easier to deform than the second channel 1022, and thus easy to deform together with the deformation of the first channel 1021.

In this embodiment, in order to allow the covered stent 100 to be more easily compressed and have a smaller compressed and folded volume when mounted on a delivery device, the proximal support corrugated rings 1011, the distal support corrugated rings 1031, and first corrugated rings 1023 may be sutured onto a surface of the first covering membrane in a suturing manner. Specifically, during suture, at least crests, facing a proximal end, of the proximal support corrugated rings 1011, the distal support corrugated rings 1031, and the first corrugated rings 1023 are not sutured onto the first covering membrane. In this way, when the covered stent 100 is folded and compressed, since the crests are not constrained by suturing, and other suture points can slightly displace to adapt to the folding of the covering membrane and the deformation of the corrugated rings of the stent, such that the covered stent is better folded. Further, non-sutured crests can reduce a suture ratio of the covering membrane, so the covering membrane can have improved flexibility to adapt to blood vessels with more complex curvatures.

In this embodiment, referring to FIG. 2, the aneurysm sac section 102 includes a plurality of first corrugated rings 1023 arranged at intervals in the axial direction, and the first corrugated rings 1023 are also Z-shaped annular corrugated rings; in order to enable the aneurysm sac section 102 to maintain good flexibility, a number of waves of the plurality of first corrugated rings 1023 is configured to be the same, and the crests and/or troughs of adjacent first corrugated rings 1023 are arranged opposite to each other. Herein, the number of the waves is configured to be the same and the crests and/or troughs are opposite to each other, that is, the adjacent first corrugated rings 1023 are arranged substantially parallel, such that the adjacent first corrugated rings 1023 have uniform intervals and are connected only through the covering membrane, thereby achieving good flexibility and helping the aneurysm sac section 102 to better adapt to a curved morphology of a blood vessel. Preferably, the number of the waves of the first corrugated rings 1023 may also be equal to a number of waves of the proximal support corrugated rings 1011, and the crests and/or troughs are arranged opposite to each other, such that the aneurysm sac section 102, the proximal section 101, and a connection portion between the aneurysm sac section 102 and the proximal section 101 equally maintain good flexibility.

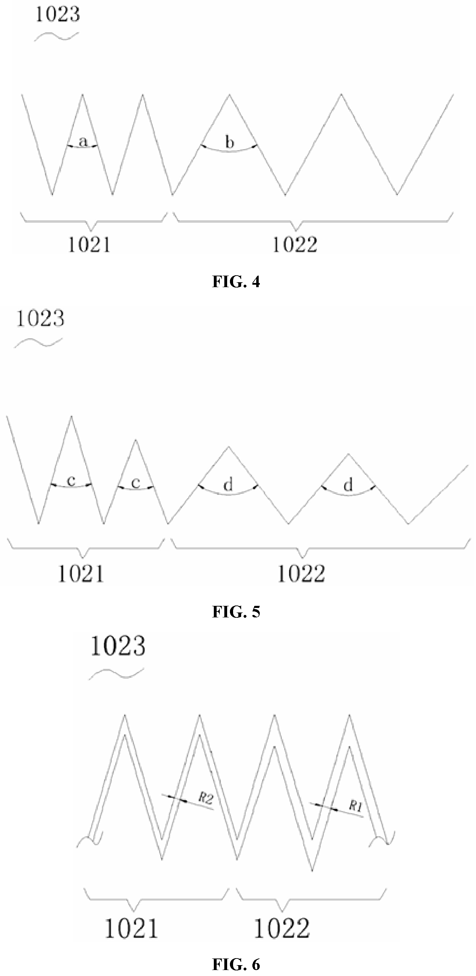

In this embodiment, referring to FIG. 4 and FIG. 5, in order to allow the total support strength of the first channel 1021 and the embedded stent 20 to be lower than the support strength of the second channel 1022, a wave angle (angle a in the figure) of the same first corrugated ring 1023, located at the second channel 1022, of the aneurysm sac section 102 is larger than a wave angle (angle b in the figure) of the same first corrugated ring 1023 located at the first channel 1021. This is because a waveform with a larger wave angle requires greater force to deform the corrugated ring than a waveform with a smaller wave angle, and the support strength of the waveform with the larger wave angle is also higher than that of the waveform with the smaller wave angle. In some embodiments, in order to create a distinction in wave angles, the adjacent first corrugated rings 1023 have the same number of waves, and the crests and/or troughs are arranged opposite to each other, so that a wave height of a waveform in a portion corresponding to the second channel 1022 may be smaller than a wave height of a waveform in a portion corresponding to the first channel 1021, and a wave angle (angle d in the figure) of the waveform in the portion corresponding to the second channel 1022 is larger than a wave angle (angle c in the figure) of the waveform in the portion corresponding to the first channel 1021. In another embodiment, wave heights of all waveforms of the first corrugated ring 1023 may gradually increase from the second channel 1022 to the first channel 1021, while wave angles may gradually decrease, to form a structure with gradually reduced support strength. In this way, the gradual reduction of the support strength may prevent a sudden large difference in support strength of the aneurysm sac section 102 at a junction of the second channel 1022 and the first channel 1021, thereby avoiding unpredictable and undesirable deformation of the aneurysm sac section 102 at this position.

In other embodiments, referring to FIG. 6, in order to allow the total support strength of the first channel 1021 and the embedded stent 20 to be lower than the support strength of the second channel 1022, a wire diameter R1 of a portion, located at the second channel 1022, of the first corrugated ring 1023 may be larger than a wire diameter R2 of a portion, located at the first channel 1021, of the first corrugated ring 1023. When the first corrugated ring with the larger wire diameter deforms, force required to resist the rigidity of a material itself is greater. Therefore, under the same force, a deformation amount of the corrugated ring with the larger wire diameter is less than a deformation amount of the corrugated ring with the smaller wire diameter, and the corrugated ring with the larger wire diameter has higher support strength. In some embodiments, small-diameter braiding wires and large-diameter braiding wires may be connected together through connecting members or fastening elements, and after connection, they are respectively placed in the first channel 1021 and the second channel 1022 of the aneurysm sac section 102. Alternatively, after the first corrugated ring 1023 is integrally formed by cutting, the wire diameter of the first corrugated ring in the portion corresponding to the first channel 1021 may be reduced in a chemical etching or physical polishing manner, or the wire diameter of the first corrugated ring 1023 may be gradually reduced from the portion corresponding to the second channel 1022 to the portion corresponding to the first channel 1021.

To allow the support strength of the embedded stent 20 and the first channel 1021 of the aneurysm sac section 102 to be lower than the support strength of the second channel 1022, the embedded stent 20 is formed by braiding small-diameter metal braiding wires. In this embodiment, a wire diameter d of the embedded stent 20 is less than one-half of a wire diameter D of the first corrugated ring 1023 in the portion corresponding to the second channel 1022, that is, d is less than one-half of D, and a wire diameter M of the first corrugated ring 1023 in the portion corresponding to the first channel 1021 is also less than one-half of the wire diameter D of the first corrugated ring 1023 in the portion corresponding to the second channel 1022, thereby ensuring that a sum of the support strength of the embedded stent 20 and the first channel 1021 is always lower than the support strength of the second channel 1022. However, according to the present invention, the wire diameters of the embedded stent 20 and the first corrugated ring 1023 in the portion corresponding to the first channel 1021 are not be configured only as described above, and those skilled may adjust the wire diameter of the embedded stent 20 and the wire diameter of the first corrugated ring 1023 in the portion corresponding to the first channel 1021 according to actual stent requirements, to ensure that the sum of the support strength of the embedded stent and the first channel is lower than the support strength of the second channel 1022.

The support strength of the aneurysm sac section 102 located at the proximal end and/or the distal end may also be higher than the support strength at a middle position. In one embodiment, the support strength of the first corrugated rings 1023, located at the proximal end and the distal end, of the aneurysm sac section 102 is higher than the support strength of the first corrugated ring 1023 at the middle position. This is because when an aneurysm sac is formed in a blood vessel, a junction between the aneurysm sac and a normal blood vessel is typically a severely compressed position, and the first corrugated rings 1023 at the proximal end and the distal end of the aneurysm sac section 102 are configured to have higher support strength to better resist compression from the junction between the blood vessel and the aneurysm sac; and further, the first corrugated ring at the middle position is configured to have lower support strength to enable the aneurysm sac section 102 to have certain flexibility and better adapt to a morphology of the blood vessel. In other embodiments, the support strength of the first corrugated ring 1023 located at the proximal end of the aneurysm sac section 102 may be only configured to be higher than the support strength of the first corrugated ring 1023 at the middle position, such that at least the support strength of the stent located at a blood inflow position may be ensured. In order to achieve the above-described change in support strength, the wire diameter or wave angle of the first corrugated ring 1023 at the proximal end of the aneurysm sac section 102 may be greater than the wire diameter or wave angle of the first corrugated ring 1023 at the middle position.

In this embodiment, the support strength is specifically manifested as a deformation amount of a whole tubular lumen (the first channel 1021 and the second channel 1022) of the whole covered stent 100 after the aneurysm sac section 102 is compressed, that is, outer walls of the first channel 1021 and the second channel 1022 are compressed using the same force (this force needs to deform both the first channel 1021 and the second channel 1022), and radial cross-sectional areas of the first channel 1021 and the second channel 1022 after compression can be measured and calculated. Herein, the stent with a larger total cross-sectional area measured after compression has higher support strength, and the stent with a smaller total cross-sectional area has lower support strength. In some embodiments, the first channel 1021 and the second channel 1022 which are independently separated can be compressed by a flat panel dynamometer, and force required by the same deformation amount through compression is measured. If the measured force is greater, the support strength is higher, and if the measured force is smaller, the support strength is lower. In this embodiment, the force measured on the second channel 1022 is always greater than the force measured on the first channel 1021.

### Embodiment 2:

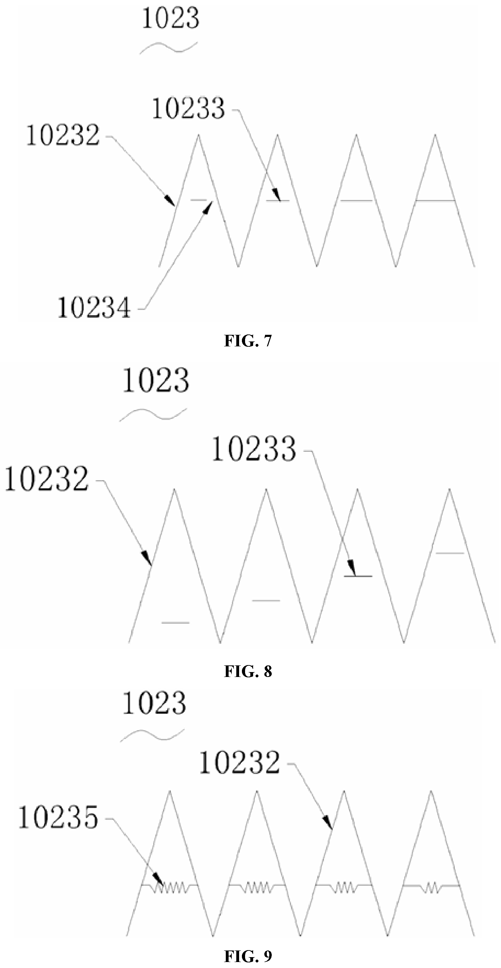

In this embodiment, referring to FIG. 7 to FIG. 9, structures of a main stent 10 and an embedded stent 20 are substantially the same as those in Embodiment 1, and a difference lies in that a first corrugated ring 1023 includes a plurality of first wave struts 10232, and the plurality of first wave struts 10232 are connected end to end at an angle, to form a Z-shaped or M-shaped annular corrugated ring, wherein a support member 10233 is arranged between adjacent first wave struts 10232, and the support member 10233 is connected to a first covering membrane 104 between two adjacent first wave struts 10232 in a suturing or adhesive bonding manner, such that when the two first wave struts 10232 are subjected to compressive force and deform, the two first wave struts 10232 perform a mutually approaching compressive motion around their wave angle, and when they move to the support member 10233, two sides of the support member 10233 respectively abut against the two first wave struts 10232, to prevent the two first wave struts 10232 from continuously moving for compression, thereby limiting a deformation degree of a waveform to provide supporting force. To allow the support strength of the first corrugated ring 1023 in the portion corresponding to a second channel 1022 to be higher than the support strength of the first corrugated ring in the portion corresponding to a first channel 1021, a plurality of support members 10233 are configured such that a compressible distance between the first wave struts 10232 in the portion corresponding to the second channel 1022 is smaller than a compressible distance between the first wave struts 10232 in the portion corresponding to the first channel 1021. This is because the compressible distance between the two adjacent first wave struts 10232 is smaller, the deformation degree of the waveform is reduced, and the support performance of the first corrugated ring 1023 at this waveform position is better.

Referring to FIG. 7 and FIG. 8, in one embodiment, in order to achieve the above effect, a gap 10234 may be formed between the support member 10233 and the adjacent first wave strut 10232, and the purpose of forming the gap 10234 is to provide movable deformation space between the first wave strut 10232 and the support member 10233. The space is larger, the gap 10234 is larger, the deformation degree of the waveform is greater, and the support strength is lower. Conversely, the gap 10234 is smaller, the deformation degree of the waveform is smaller, and the support strength is higher. Therefore, by configuring the gap 10234 between the support member 10233 in the portion corresponding to the second channel 1022 and the adjacent first wave strut 10232 to be smaller than the gap 10234 between the support member 10233 in the portion corresponding to the first channel 1021 and the adjacent first wave strut 10232, the support strength obtained by the first corrugated ring 1023 in the portion corresponding to the second channel 1022 can be higher than the support strength obtained by the first corrugated ring in the portion corresponding to the first channel 1021. In this way, when the first corrugated ring 1023 is subjected to compressive force, the first wave struts 10232 deform in a mutual approaching manner, and when the first wave struts 10232 in the portion corresponding to the second channel 1022 abut against the support member 10233, the first wave struts 10232 in the portion corresponding to the first channel 1021 can still continue to deform, such that a deformation amount of the aneurysm sac section 102 in the portion corresponding to the first channel 1021 is greater than a deformation amount of the aneurysm sac section in the portion corresponding to the second channel 1022.

Referring to FIG. 7 and FIG. 8, the support member 10233 may have a linear structure having two ends, and in order to prevent the two ends of the linear-structure support member 10233 from piercing a covering membrane and scratching a blood vessel, the two ends may be bent into a ring or configured as anti-damage ends, etc., which is not shown in the figures. It may be understood that a plurality of linear-structure support members 10233 may be support members 10233 with the same length, and are arranged at different axial positions between the adjacent first wave struts 10232. For example, in the portion corresponding to the second channel 1022, the support members 10233 with the same length are arranged close to wave angles, to provide smaller gaps 10234. It may also be understood that the plurality of linear-structure support members 10233 may be support members 10233 with different lengths, and are arranged at the same axial positions between the adjacent first wave struts 10232. For example, the length of the support members 10233 in the portion corresponding to the second channel 1022 is greater than the length of the support members 10233 in the portion corresponding to the first channel 1021, to provide smaller gaps 10234.

In another embodiment, referring to FIG. 9, the support member 10233 is an elastic support member 10235, wherein two sides of the elastic support member 10235 are respectively connected to the adjacent first wave struts 10232. In this way, a mutual approaching deformation degree of the two adjacent first wave struts 10232 is determined by an elastic deformation degree of the elastic support member 10235. When the elastic deformation degree of the elastic support member 10235 is great, that is, an elastic modulus is small, the mutual approaching deformation degree of the two adjacent first wave struts 10232 is great, and the support strength that can be provided is low; and when the elastic deformation degree of the elastic support member 10235 is small, that is, the elastic modulus is great, the mutual approaching deformation degree of the two adjacent first wave struts 10232 is small, and the support strength that can be provided is high. In this way, by configuring the elastic modulus of the elastic support member 10235 in the portion corresponding to the second channel 1022 to be greater than the elastic modulus of the elastic support member 10235 in the portion corresponding to the first channel 1021, the elastic support member 10235 having the greater elastic modulus provides greater supporting force in the portion corresponding to the second channel 1022, whereas the elastic support member 10235 having the smaller elastic modulus provides smaller supporting force in the portion corresponding to the first channel 1021.

In some embodiments, the elastic support member 10235 may be a spring structure, two sides of the spring structure are respectively connected to the first wave struts 10232 on two sides, and an elastic modulus of the spring structure in the portion corresponding to the second channel 1022 is configured to be greater than an elastic modulus of the spring structure in the portion corresponding to the first channel 1021.

In some other embodiments, referring to FIG. 9, the elastic support member 10235 may be an elastic connecting member that has a spring element in the middle and connecting structures on two sides, wherein a total length of the middle spring element can be configured to limit a deformation distance of the first wave struts 10232 on two sides. For example, the length of the spring element of the elastic connecting member in the portion corresponding to the second channel 1022 is configured to be smaller than the length of the spring element of the elastic connecting member in the portion corresponding to the first channel 1021, and a deformation degree that can be provided by the shorter spring element is smaller than a deformation degree that can be provided by the longer spring element. In this way, under the same force, the shorter spring element reaches a deformation limit before the longer spring element, to provide stable supporting force.

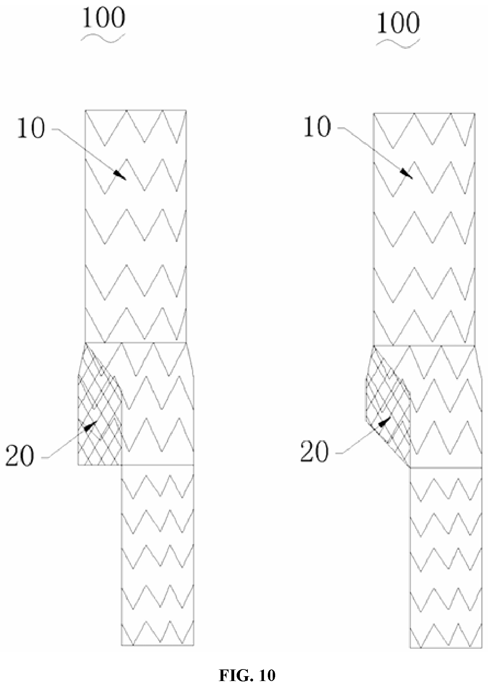

In other embodiments, referring to FIG. 10, by allowing an axial length of an aneurysm sac section 102 in the portion corresponding to the first channel 1021 and the embedded stent 20 to be smaller than an axial length in the portion corresponding to the second channel 1022, a support portion of the aneurysm sac section is decreased when the aneurysm sac section 102 is compressed in a radial direction, and an area that can withstand compression is small. In this way, the support strength of the aneurysm sac section 102 in the portion corresponding to the first channel 1021 may be lower than the support strength in the portion corresponding to the second channel 1022.

In some embodiments, referring to FIG. 10, a distal opening of the embedded stent 20 and an opening 1024 may be configured as oblique openings, and the oblique openings are arranged away from the second channel 1022. Moreover, the arrangement of the oblique openings can also increase an entry opening when an internal iliac stent is implanted into the embedded stent 20, to reduce an entry difficulty. A proximal opening of the embedded stent 20 is also configured as an oblique opening to increase a blood inflow receiving area.

In one embodiment, the support member 10233 is at least partially provided with a developing structure (not shown in the figure). When the support member 10233 is provided with the developing structure, the support member 10233 can be configured as a shape and structure with a marking identification function according to requirements, such as a letter-shaped support member 10233 or a figure-shaped support member 10233. The support member 10233 may also be a developing structure entirely, and a preferable developing structure may be a tantalum wire or gold wire.

### Embodiment 3

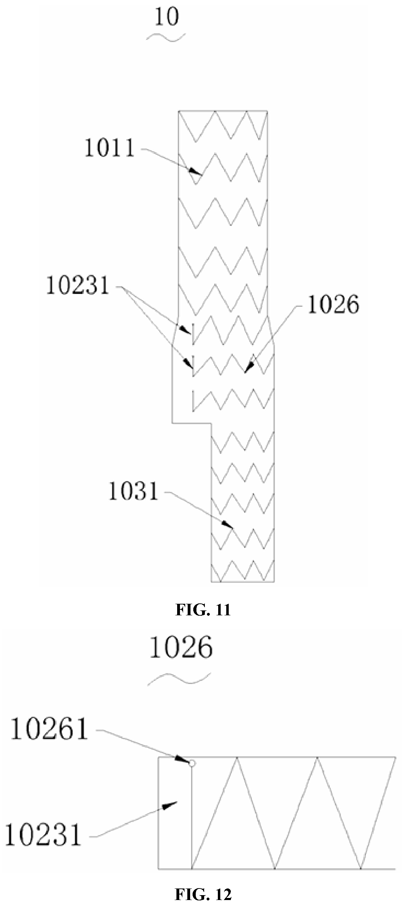

In this embodiment, referring to FIG. 11 and FIG. 12, structures of a main stent 10 and an embedded stent 20 are substantially the same as those in Embodiment 1, and a difference lies in that a first corrugated ring 1023 is provided with an opening 10231 at least in a portion corresponding to a first channel 1021. It may be understood that the first corrugated ring 1023 is provided with the opening 10231 to form a C-shaped corrugated ring 1026, the first corrugated ring 1023 does not provide supporting force at the opening 10231, the opening 10231 is formed at the first channel 1021, an aneurysm sac section 102 is only covered with a covering membrane in the portion corresponding to the first channel 1021, and is not supported by the first corrugated ring 1023, supporting force for the first channel 1021 is provided by the embedded stent 20, while the support strength of the embedded stent 20 is lower than the support strength of the first corrugated ring 1023, such that the support strength of the aneurysm sac section 102 in the portion corresponding to the first channel 1021 is lower than the support strength in the portion corresponding to a second channel 1022.

In one embodiment, the opening 10231 only covers a connection portion of the embedded stent 20 and the covering membrane of the aneurysm sac section 102. Two ends of the C-shaped corrugated ring 1026 at the opening are connected to two sides of the connection portion of the embedded stent 20 and the covering membrane of the aneurysm sac section 102. Thus, the portion corresponding to the first channel 1021 of the aneurysm sac section 102 that is connected only to the embedded stent 20 is not supported by the first corrugated ring 1023. In this way, when the aneurysm sac section 102 is subjected to compressive force, the portion corresponding to the first channel 1021 that is connected to the embedded stent 20 deforms first due to lower support strength, to effectively avoid excessive deformation of the second channel 1022.

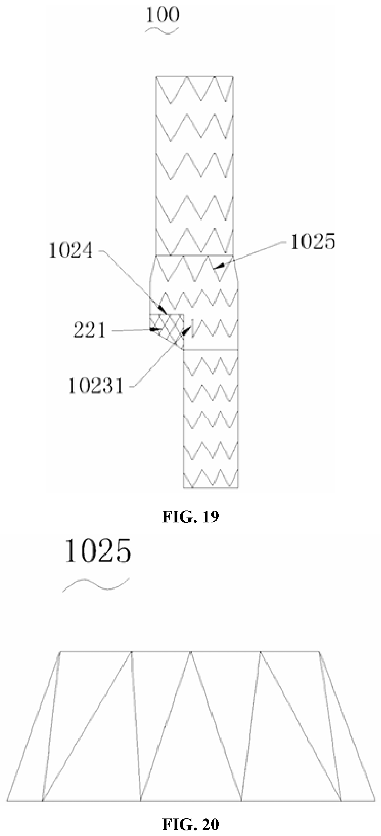

Further referring to FIG. 20, two end portions of the C-shaped corrugated ring 1026, which are located at the opening 10231, are wrapped around to form a circular ring structure 10261 or anti-damage ends, and the circular ring structure 10261 can accommodate end portions of a metal wire for braiding the C-shaped corrugated ring 1026, thereby preventing the sharp end portions from piercing the covering membrane and scratching a blood vessel.

### Embodiment 4

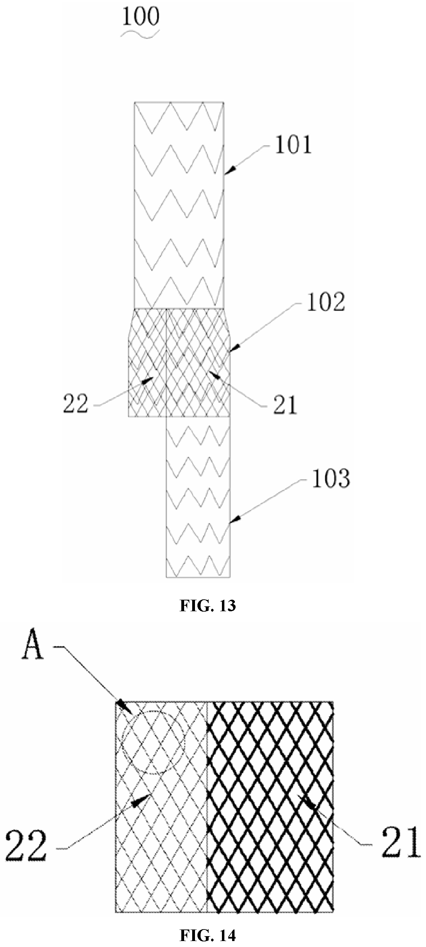

In this embodiment, referring to FIG. 13, structures of a main stent 10 and an embedded stent 20 are substantially the same as those in Embodiment 1, and a difference lies in that the embedded stent 20 in an aneurysm sac section 102 includes a first embedded stent 21 and a second embedded stent 22 which are arranged in a radial direction, wherein the first embedded stent 21 is in communication with a distal section 103, a distal end of the aneurysm sac section 102 is provided with an opening 1024, and a distal opening of the second embedded stent 22 is in communication with the opening 1024. The aneurysm sac section 102 includes a first channel 1021 and a second channel 1022, the second channel 1022 accommodates the first embedded stent 21, and the first channel 1021 accommodates the second embedded stent 22.

The second channel 1022 and the first channel 1021 are separated by embedding the first embedded stent 21 and the second embedded stent 22 to form blood flowing lumens, and the first embedded stent 21 and the second embedded stent 22 can provide support to maintain channel forms and avoid compression-induced occlusion. The first embedded stent 21 is arranged in the second channel 1022, and when subjected to compression, the first embedded stent 21 of the second channel 1022 can have supporting force against the second embedded stent 22 of the first channel 1021, such that the covered stent 100 of the present invention can provide a pathway with a good form when an internal iliac stent is implanted into the first channel 1021, and can also avoid occlusion of the second channel 1022 caused by excessive compression on the second channel 1022 by the first channel 1021, thereby better maintaining entire forms and patency of the two channels. A blood inflow opening of the aneurysm sac section 102 located at the proximal section 101 and a blood outflow opening located at the distal end are occupied by the proximal openings and the distal openings of the first embedded stent 21 and the second embedded stent 22, and when blood flows from the proximal section 101 into the aneurysm sac section 102, it is shunted by the first embedded stent 21 and the second embedded stent 22.

In this embodiment, referring to FIG. 14 to FIG. 16, in order to ensure an deployment form and patency of the second channel 1022 when the aneurysm sac section 102 is compressed or the stent is deployed, the support strength of a portion, located at the second channel 1022, of the entire aneurysm sac section 102 of the covered stent 100 is configured to be higher than the support strength of that of a portion located at the first channel 1021. That is, on a premise of uniform support strength of the aneurysm sac section 102, it can be achieved by configuring the support strength of the first embedded stent 21 to be higher than the support strength of the second embedded stent 22; or on a premise that the support strength of the first embedded stent 21 is equal to the support strength of the second embedded stent 22, it can be achieved by configuring the support strength of the second channel 1022 of the aneurysm sac section 102 to be higher than the support strength of the first channel 1021; or it can be achieved by configuring the support strength of the first embedded stent 21 to be higher than the support strength of the second embedded stent 22 and configuring the support strength of the second channel 1022 of the aneurysm sac section 102 to be higher than the support strength of the first channel 1021.

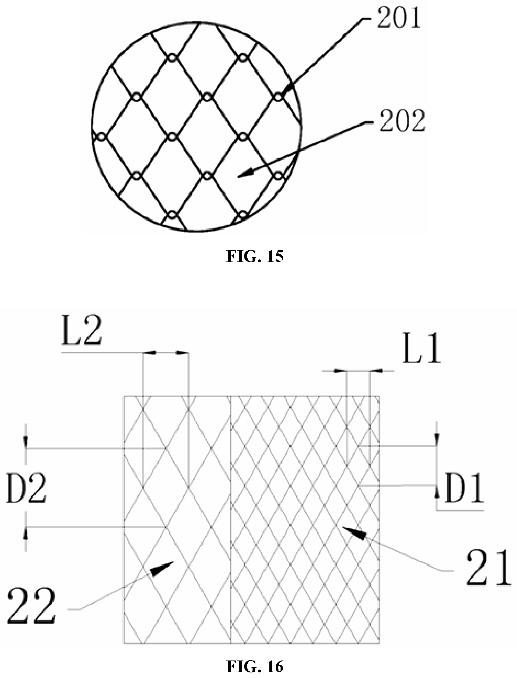

In this embodiment, referring to FIG. 14 and FIG. 15, the first embedded stent 21 and the second embedded stent 22 each have a mesh main body 201 and a surface second covering membrane 202, and the aneurysm sac section 102 of the main stent 10 includes a plurality of first corrugated rings 1023 arranged at intervals in an axial direction. The mesh main body 201 can provide better ductility, and more flatly spread surface second covering membranes 202 of the first embedded stent 21 and the second embedded stent 22, to prevent local portions from collapsing due to compression, and the mesh main body also can provide more support sites to abut against the internal iliac stent during implantation of the internal iliac stent, to increase frictional force. By adjusting wire diameters of the first embedded stent 21 and the second embedded stent 22, and adjusting the wire diameter of the first corrugated ring 1023 located at the second channel 1022 and the wire diameter of the first corrugated ring 1023 located at the first channel 1021, the covered stent 100 can be configured such that the portions of the aneurysm sac section 102 corresponding to the first channel 1021 and the second channel 1022 have different support strengths. It may be understood that a larger wire diameter of the stent can provide higher support performance; and therefore, the first embedded stent 21 may have a wire diameter greater than that of the second embedded stent 22, and/or the wire diameter of the first corrugated ring 1023 in the second channel 1022 may be greater than the wire diameter of the first corrugated ring 1023 in the first channel 1021.

Referring to FIG. 14, the wire diameter of the first embedded stent 21 is allowed to be larger than the wire diameter of the second embedded stent 22, and the wire diameter of the first corrugated ring 1023 located at the second channel 1022 is allowed to be larger than the wire diameter of the first corrugated ring 1023 located at the first channel 1021. Since the wire diameter of the first corrugated ring 1023 at the first channel 1021 is small, when the aneurysm sac section 102 is subjected to compressive force, the first corrugated ring 1023 at the first channel 1021 deforms before the first corrugated ring 1023 at the second channel 1022, to first affect deformation of the second embedded stent 22 in the first channel 1021. Since the wire diameter of the second embedded stent 22 is smaller than the wire diameter of the first embedded stent 21, the second embedded stent 22 deforms before the first embedded stent 21, the support strength on a single-layer stent is dispersed to an inner-layer stent and an outer-layer stent, and the support strength on a single stent is not too high. In addition, the wire diameters of the first corrugated ring 1023 and a second internal iliac stent at the first channel 1021 of the aneurysm sac section 102 are reduced simultaneously, an excessively large difference in wire diameter of the single stent can be avoided, and the wire diameters of the two stents are not reduced excessively, thereby avoiding problems that the configuration of an excessively small wire diameter causes the stent to hardly maintain a supporting effect and the configuration of an excessively large wire diameter causes poor flexibility.

### Embodiment 5

In this embodiment, referring to FIG. 16, structures of a main stent 10 and an embedded stent 20 are substantially the same as those in Embodiment 1 and Embodiment 4, and a difference lies in that in this embodiment, by allowing a grid density of a first embedded stent 21 to be greater than a grid density of a second embedded stent 22, the support strength of a portion, located at a second channel 1022, of an entire aneurysm sac section 102 of the covered stent 100 is configured to be higher than the support strength of a portion located at a first channel 1021. Specifically, the first embedded stent 21 and the second embedded stent 22 each include a mesh main body 201, and the mesh main body 201 has a plurality of grid structures; a higher grid density means a greater number of required braiding wires; and a smaller area of a single grid can withstand a higher pressure, and the higher grid density can provide higher support strength.

In this embodiment, continuing to refer to FIG. 16, the first embedded stent 21 and the second embedded stent 22 each have a braided diamond grid structure, the first embedded stent 21 has a first diamond grid, the second embedded stent 22 has a second diamond grid, the first diamond grid and the second diamond grid each have an upper vertex and a lower vertex in an axial direction, and a left vertex and a right vertex in a radial direction; and a distance between the upper vertex and the lower vertex of the first diamond grid is D1, and a distance between the left vertex and the right vertex is L1. A distance between the upper vertex and the lower vertex of the second diamond grid is D2, and a distance between the left vertex and the right vertex is L2. Preferably, both D1 and L1 may be smaller than D2 and L2, such that an area occupied by a single first diamond grid is smaller than an area occupied by a single second diamond grid. Therefore, under the same stent deployment area, the first embedded stent 21 has a higher grid density and provides higher support strength than the second embedded stent 22.

In another embodiment, continuing to refer to FIG. 16, at least L1 of the first diamond grid can be made smaller than L2 of the second diamond grid, such that the density of the first diamond grid is changed only in a circumferential direction of the first embedded stent 21. In this way, the density of the first diamond grid can be increased at least in a radial direction, thereby achieving an effect of improving the support strength in the radial direction.

In one embodiment, a wave angle of a first corrugated ring 1023 of the aneurysm sac section 102 in the portion corresponding to the second channel 1022 is greater than a wave angle of a first corrugated ring 1023 in the portion corresponding to the first channel 1021; and in another embodiment, wave heights of all waveforms of the first corrugated ring 1023 may gradually increase from the portion corresponding to the second channel 1022 to the portion corresponding to the first channel 1021, wave angles may gradually decrease, and a structure with gradually reduced support strength is formed. In this way, the gradual reduction of the support strength may prevent a sudden, large difference in support strength of the aneurysm sac section 102 at a junction of the second channel 1022 and the first channel 1021, thereby avoiding unpredictable and undesirable deformation of the aneurysm sac section 102 at this position. By simultaneously making the grid density of the first embedded stent 21 greater than the grid density of the second embedded stent 22 and the wave angle of the first corrugated ring 1023 in the portion corresponding to the second channel 1022 greater than the wave angle of the first corrugated ring 1023 in the portion corresponding to the first channel 1021, and also configuring changes in support strength on the first corrugated ring 1023 of the aneurysm sac section 102, the first embedded stent 21, and the second embedded stent 22, it is possible to avoid the problem of poor stent flexibility at specific portions which may be caused by configuring a change in the support strength only on the first embedded stent 21 and the second embedded stent 22 or only on the first corrugated ring 1023 of the aneurysm sac section 102.

It may be understood that by configuring the grid density of the first embedded stent 21 to be greater than the grid density of the second embedded stent 22, and/or configuring the wave angle of the first corrugated ring 1023 in the portion corresponding to the second channel 1022 to be greater than the wave angle of the first corrugated ring 1023 in the portion corresponding to the first channel 1021, the support strength of the aneurysm sac section 102 of the covered stent 100 in the portion corresponding to the second channel 1022 may be configured to be greater than the support strength of the aneurysm sac section 102 in the portion corresponding to the first channel 1021.

### Embodiment 6

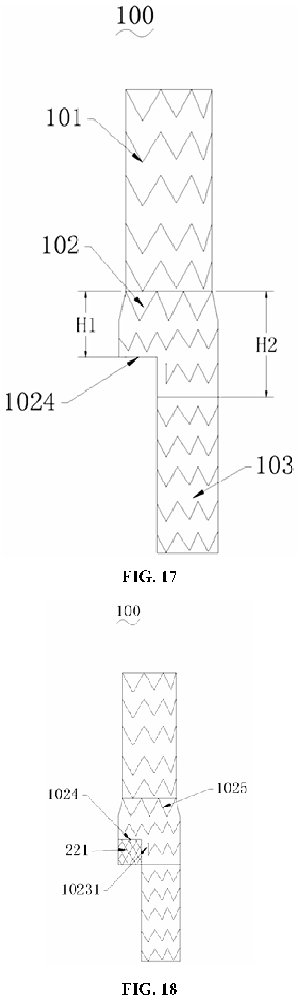

In this embodiment, referring to FIG. 17 to FIG. 19, structures of a main stent 10 and an embedded stent 20 are substantially the same as those in Embodiment 1 and Embodiment 4 to Embodiment 5, and a difference lies in that the support strength of an aneurysm sac section 102 in the portion corresponding to a first channel 1021 and in the portion corresponding to a second channel 1022 is changed by configuring different axial lengths (H1 and H2 in FIG. 17) for the first channel 1021 and the second channel 1022 of the aneurysm sac section 102. Specifically, the axial length H2 of the second channel 1022 is greater than the axial length H1 of the first channel 1021, the aneurysm sac section 102 has the axial length H2 in the portion corresponding to the second channel 1022 and the axial length H1 in the portion corresponding to the first channel 1021, and H2 is greater than H1, such that when the second channel 1022 of the aneurysm sac section 102 is compressed, a pressure bearing length and a contact area are greater than a pressure bearing length and a contact area of the first channel 1021, thereby better dispersing the pressure, providing better support performance, and improving support strength. Further referring to FIG. 18 and FIG. 19, a distal end of the second embedded stent 22 includes an exposed section 221, the exposed section 221 passes through an opening 1024, and the exposed section 221 passes through the first channel 1021, such that the first channel 1021 of the covered stent 100 also has distribution of different support strengths; and at a deployment end of an internal iliac stent, support is provided solely by a single layer of the second embedded stent 22, such that improved flexibility is achieved at this position, thereby facilitating the insertion of the internal iliac stent. Further, a distal opening of the exposed section 221 is a flat opening or an oblique opening; when it is configured as the flat opening, the exposed section 221 can have more attachment support positions with the implanted internal iliac stent, such that the long-term stability of the implanted internal iliac stent can be effectively improved; when it is configured as the oblique opening, the opening is away from the second channel 1022, such that an entry for the internal iliac stent can be enlarged without increasing a diameter of the second embedded stent 22, thereby facilitating faster and easier deployment of the internal iliac stent; and the enlarged entry can also improve the accuracy of deployment of the internal iliac stent. In some embodiments, the exposed section 221 is attached to the adjacent second channel 1022, and the exposed section 221 may be a free end and may be separated from the second channel 1022. Such configuration can prevent the internal iliac stent from being subjected to an excessive pressure at this position when the internal iliac stent is implanted and extends to an internal iliac blood vessel, thereby avoiding folding and occlusion. The exposed section 221 may also be fixedly connected to a side wall of the second channel 1022 in a suturing or adhesive bonding manner, thereby avoiding swinging and facilitating the employment of the internal iliac stent.

Referring to FIG. 18 to FIG. 19, because the second embedded stent 22 passes through the opening 1024 of the aneurysm sac section 102, and only the single-layer stent is arranged at the opening 1024, support performance at this position is greatly reduced compared to that at other positions. In order to ensure that the aneurysm sac section 102 is easier to deform in the portion corresponding to the first channel 1021 than in the portion corresponding to the second channel 1022 when the aneurysm sac section 102 of the covered stent 100 of the present invention is compressed, and to prevent compression-induced occlusion of the entry of the exposed section 221 from weakening support performance of the stent at a portion, close to the exposed section 221, of the second channel 1022, at least a first corrugated ring 1023, at least in a portion corresponding to the same axial position as the exposed section 221, is provided with an opening 10231 in the second channel 1022, to form a C-shaped corrugated ring 1026, and the opening 10231 faces the exposed section 221. In this way, the support performance at a position, in contact with the exposed section 221, of the second channel 1022 is effectively weakened, and when the entry of the internal iliac stent of the exposed section 221 is compressed, a certain amount of deformation can be generated at a position abutting against the second channel 1022 to buffer compressive force, thereby preventing the entry from being further compressed.

As in Embodiment 3, two end portions of the C-shaped corrugated ring 1026, which are located at the opening 10231, are wrapped around to form a circular ring structure, and the circular ring structure can accommodate end portions of a metal wire for braiding the C-shaped corrugated ring 1026, thereby preventing the sharp end portions from piercing a covering membrane and scratching a blood vessel.

In this embodiment, referring to FIG. 20, in order to enable the covered stent 100 to better adapt to the morphology of a diseased blood vessel, a proximal corrugated ring 1025 is arranged at a proximal end of the aneurysm sac section 102, and the proximal corrugated ring 1025 at the proximal end of the aneurysm sac section 102 is configured such that a diameter of the proximal end is smaller than a diameter of a distal end, thereby providing a tapered angle for the proximal corrugated ring 1025 and forming a narrow-proximal and wide-distal stent structure. In this way, the aneurysm sac section 102 of the covered stent 100 forms a gradually varying transition structure form wide to narrow at the junction with a proximal section 101, to accommodate a diameter change from a normal blood vessel to a diseased blood vessel, and to enhance anchoring performance of the proximal section 101 in the blood vessel, thereby improving stability of the covered stent 100 after implantation. In this embodiment, in order to allow both the first channel 1021 and the second channel 1022 of the aneurysm sac section 102 to have high patency, a radial dimension of the aneurysm sac section 102 is configured to be greater than those of the proximal section 101 and a distal section 103. In addition, the arrangement of a first embedded stent 21 and the arrangement of the second embedded stent 22 can ensure patency of the first channel 1021 and an external iliac channel. An aneurysm sac blood vessel has a dimensional change from large to small like a proximal blood vessel, such that the proximal corrugated ring arranged can match this structure, surface attachment of the covered stent 100 of the present invention can be enhanced, and the sent is prevented from swinging in the blood vessel and also prevented from migrating and displacing in the blood vessel.

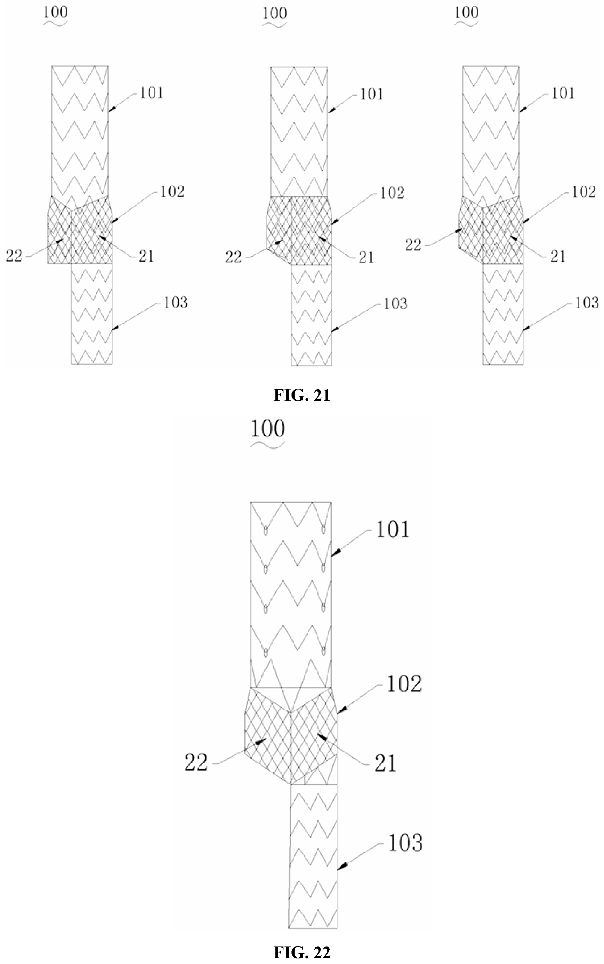

In other embodiments, referring to FIG. 21, proximal openings and distal openings of the first embedded stent 21 and the second embedded stent 22 can be respectively configured as flat openings or oblique openings. For example, the proximal opening and the distal opening of the second embedded stent 22 are configured as parallel oblique openings, and the proximal opening of the first embedded stent 21 is configured as an oblique opening. In this way, the second embedded stent 22 may be further allowed to form an unstable parallelogram structure to have staggered support points in a radial direction, and is easy to deform when compressed to reduce the support strength, while the configuration of the proximal openings of the first embedded stent 21 and the second embedded stent 22 as the oblique openings enables formation of larger blood inflow openings to smoothly receive blood from the proximal section.

In this embodiment, a first covering membrane 104 covers a surface of the main stent 10, second covering membranes 202 cover surfaces of the first embedded stent 21 and the second embedded stent 22, and the support strength of the first covering membrane 104 is higher than the support strength of the second covering membrane 202, wherein the first covering membrane 104 is a PET membrane, and the second covering membranes 202 are ePTFE membranes. Herein, a change in support strength of the first covering membrane 104 and the second covering membranes 202 is primarily attributed to inherent properties of materials. The tensile strength of the PET membrane adopted by the first covering membrane 104 usually ranges from 50 to 200 MPa, and the first covering membrane has good impact resistance, while the tensile strength of the ePTFE membranes adopted by the second covering membranes 202 usually ranges from 23 to 30 MPa, and the second covering membranes have poor impact resistance. Therefore, the first covering membrane 104 and the second covering membranes 202, which have different tensile strengths, are adopted to enable the main stent 10, the first embedded stent 21, and the second embedded stent 22 to have differences in support strength in an aspect of the covering membranes, to better match the stent itself for configuration of different support strengths. The purpose of configuring the ePTFE membranes for the first embedded stent 21 and the second embedded stent 22 is to reduce the impact of the covering membranes on the support strength to the greatest extent while improving blood patency.

In another embodiment, in order to enable the covered stent 100 provided in the present invention to better adapt to a diseased iliac artery blood vessel, the support strength of the distal section 103 and the proximal section 101 of the covered stent 100 is configured to be higher than the total support strength of the aneurysm sac section 102, the first embedded stent 21, and the second embedded stent 22. This is because the aneurysm sac section 102 itself has support strength, and the support strength of the covered stent 100 at this position is measured as the total support strength of the aneurysm sac section 102, the first embedded stent 21, and the second embedded stent 22 after the first embedded stent 21 and the second embedded stent 22 are arranged. The support strength of the proximal section 103 and the distal section 101 of the covered stent 100 is configured to be higher than the total support strength of the aneurysm sac section 102, the first embedded stent 21, and the second embedded stent 22, such that the covered stent 100 provides greater supporting force in a proximal blood vessel and a distal blood vessel adjacent to an iliac artery lesion, thereby enhancing its anchoring force in the blood vessels. The covered stent 100 of the present invention is firmly anchored to a release position; and the purpose of weakening the support strength of the aneurysm sac section 102 is to ensure its improved flexibility, thereby improving adaptability, better attaching the blood vessel of the aneurysm sac section 102, and avoiding poor attachment. In some other embodiments, the support strength of the distal section 103 or the proximal section 101 of the covered stent 100 is configured to be higher than the total support strength of the aneurysm sac section 102, the first embedded stent 21, and the second embedded stent 22; and higher support strength is configured at either end of the proximal section 101 or the distal section 103 to meet a requirement that at least one end has higher support and anchoring performance under a condition of good flexibility of the aneurysm sac section 102, to ensure the stability of the covered stent 100 when the covered stent is anchored in the blood vessel in the human body.

In some embodiments, this can be achieved by simultaneously reducing the support strength of the first embedded stent 21, the second embedded stent 22, and the aneurysm sac section 102, such as by reducing the overall support strength of the first embedded stent 21 and the second embedded stent 22 to less than one-half of the support strength of the proximal section 101 or the distal section 103, and by reducing the support strength of the aneurysm sac section 102 to less than one-half of the support strength of the proximal section 101 or the distal section 103.

### Embodiment 7

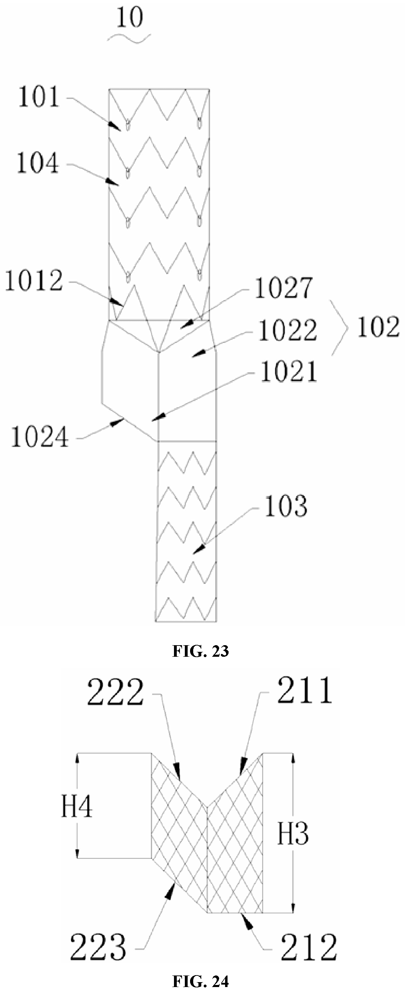

In this embodiment, referring to FIG. 22 and FIG. 23, structures of a main stent 10 and an embedded stent are substantially the same as those in Embodiment 1 and Embodiment 4 to Embodiment 6, and a difference lies in that the main stent 10 is provided with support corrugated rings at a proximal section 101 and a distal section 103, while an aneurysm sac covered section 102 is not provided with any first corrugated rings 1023, and is only covered with a covering membrane, a first embedded stent 21 and a second embedded stent 22 are arranged in a lumen of the aneurysm sac covered section 102, and support performance is provided through the first embedded stent 21 and the second embedded stent 22. The proximal section 101, the aneurysm sac covered section 102, and the distal section 103 of the main stent 10 may be connected through a first covering membrane 104, the support corrugated rings are arranged on surfaces of the proximal section 101 and the distal section 103, and only the covering membrane is arranged on the aneurysm sac covered section 102. Alternatively, the proximal section 101 and the aneurysm sac covered section 102 may be connected through a single covering membrane, and the distal section 103 may be connected to a distal end of a second channel 1022 of the aneurysm sac covered section 102 in an adhesive bonding or suturing manner. Referring to FIG. 24, the first embedded stent 21 and the second embedded stent 22 each include a mesh main body 201, and the purpose of arranging the mesh main body 201 is to achieve improved form supporting performance. Compared with the support corrugated rings, the mesh main body 201 can have greater covering membrane tension, such that even under a condition of a smaller wire diameter of a braiding wire, the mesh main body 201 can also provide greater covering membrane tension to maintain a form of a blood pathway. The aneurysm sac covered section 102 is only covered with the covering membrane, and the support strength of the covered stent 100 at the entire aneurysm sac covered section 102 can be lower than that of the proximal section 101 and the distal section 103 under a condition of smaller wire diameters of the first embedded stent 21 and the second embedded stent 22, such that the entire aneurysm sac covered section 102 can have improved flexibility while maintaining forms and patency of a first channel 1021 and the second channel 1022.

In this embodiment, referring to FIG. 23 to FIG. 26, the first embedded stent 21 and the second embedded stent 22 are connected to the first covering membrane 104 of the aneurysm sac covered section 102 in an adhesive bonding or suturing manner at least at distal openings of distal ends and proximal openings of proximal ends of the first embedded stent and the second embedded stent, wherein a blood inflow opening at a proximal end and a blood outflow opening at a distal end of the aneurysm sac covered section 102 are respectively occupied by the proximal openings and the distal openings of the first embedded stent 21 and the second embedded stent 22, such that when blood flows into the aneurysm sac covered section 102 from the proximal section 101, the blood is shunted by the first embedded stent 21 and the second embedded stent 22, to avoid endoleaks at the aneurysm sac covered section 102; and the proximal openings and the distal openings of the first embedded stent 21 and the second embedded stent 22 may be configured as flat openings or oblique openings.

Referring to FIG. 24, in order to improve the passability of blood flow and ensure that the aneurysm sac covered section 102 has improved flexibility, the proximal openings of the first embedded stent 21 and the second embedded stent 22 are configured as oblique openings, wherein the proximal end located at the first embedded stent 21 is provided with a first proximal oblique opening 211, the proximal end located at the second embedded stent 22 is provided with a second proximal oblique opening 222, the first proximal oblique opening 211 and the second proximal oblique opening 222 face each other, and the two oblique openings form a V-shaped cross section on an axial tangent plane. In this way, the double oblique openings of the first embedded stent 21 and the second embedded stent 22 can increase a receiving area of the blood inflow opening of the aneurysm sac covered section 102, such that the blood flows into the stents more smoothly. Moreover, the oblique openings allow decrease of support points of the first embedded stent 21 and the second embedded stent 22 on two sides in a radial direction, such that the support strength in the radial direction can be reduced to a certain extent, and the support strength of the covered stent 100 located at the aneurysm sac covered section 102 is lower than the support strength at the proximal section 101 and the distal section 103. In addition, generally, when the stent is bent, a large curved side is an extended portion, while a small curved side is a compressed portion. The first proximal oblique opening 211 and the second proximal oblique opening 222 are configured as opposite double oblique openings, such that when the covered stent 100 is bent, the large curved side is always located at one side with a longer oblique opening extension length of the first proximal oblique opening 211 or the second proximal oblique opening 222, and the small curved side is located at one side with a shorter oblique opening extension length. Therefore, when the covered stent is bent, oblique opening structures of the first proximal oblique opening 211 and the second proximal oblique opening 222 just match a bent structure of the stent, the side with the longer oblique opening extension length is located at the large curved side, and the side with the shorter oblique opening extension length is located at the small curved side, such that at least a portion, located at the aneurysm sac covered section, of the covered stent 100 has improved flexibility, and the embedded stents can be effectively prevented from bending in the aneurysm sac covered section 102 when the covered stent 100 is bent.

In other embodiments, the arrangement of the first proximal oblique opening 211 and the second proximal oblique opening 222 enables long-axis side walls and short-axis side walls of the first embedded stent 21 and the second embedded stent 22 to be staggered. Consequently, when the covered stent 100 in the present invention is compressed and placed into a delivery sheath, the staggered configuration of the long-axis side walls and the short-axis side walls enables the first embedded stent 21 and the second embedded stent 22 to have smaller folded volumes and to be more easily delivered into the delivery sheath.

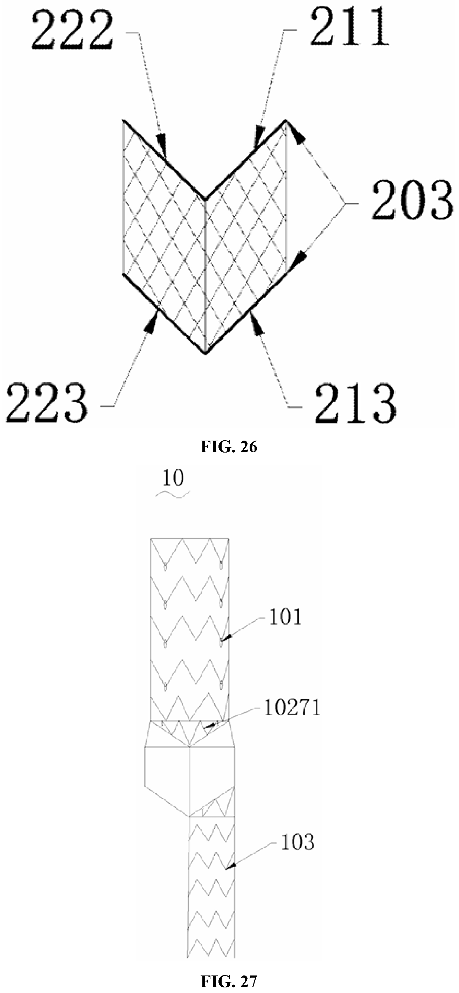

In this embodiment, referring to FIG. 24 and FIG. 26, the distal opening of the first embedded stent 21 is an oblique opening or a flat opening, the distal opening of the second embedded stent 22 is also an oblique opening or a flat opening, and the distal blood outflow opening of the aneurysm sac covered section 102 is flush with and attached to the distal openings of the first embedded stent 21 and the second embedded stent 22, and is fixed in an adhesive bonding or suturing manner. The distal opening of the second embedded stent 22 is a second distal oblique opening 223, and the distal opening of the first embedded stent 21 is a first distal oblique opening 213 or flat opening. Referring to FIG. 26, when the distal openings of the first embedded stent 21 and the second embedded stent 22 are configured as the oblique openings, the first distal oblique opening 213 and the second distal oblique opening 223 are respectively parallel to the first proximal oblique opening 211 and the second proximal oblique opening 222; and supporting force of two opposite sides of a non-rectangular parallelogram is smaller than supporting force of two opposite sides of a rectangle. In this way, the parallel oblique openings enable support positions on two sides of the first embedded stent 21 and the second embedded stent 22 in the radial direction to form a staggered structure. Therefore, under radial compressive force, part of radial pressure is converted into axial force by a special structure of the parallelogram, to effectively disperse the radial pressure to achieve an effect of reducing radial supporting force, whereby the first embedded stent 21 and the second embedded stent 22 provide channel form maintaining force, and have improved flexibility to enable the aneurysm sac covered section 102 of the covered stent 100 to have improved flexibility.

In another embodiment, referring to FIG. 24, the distal opening of the second embedded stent 22 is a second distal oblique opening 223, and the distal opening of the first embedded stent 21 is a first distal flat opening 212. Such configuration enables an axial length H3 of a side, away from the second embedded stent 22, of the first embedded stent 21 to be greater than an axial length H4 of a side, away from the first embedded stent 21, of the second embedded stent 22, such that the first embedded stent 21 has more stent supporting positions to disperse compressive force from a blood vessel, and thus has higher support strength than the second embedded stent 22.

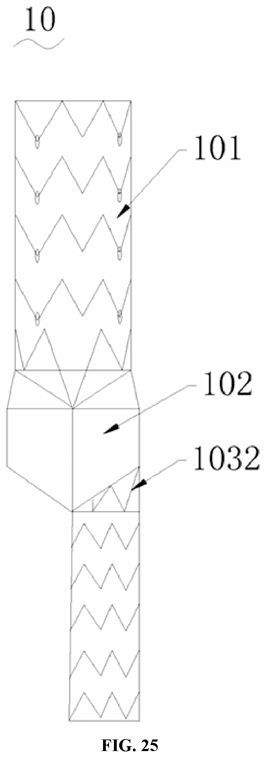

Referring to FIG. 25, when the distal opening of the first embedded stent 21 is configured as an oblique opening, the support corrugated ring at a connection portion of the distal section 103 and an aneurysm sac is configured as a rectangular corrugated ring 1032 in order to match an oblique structure of the first distal oblique opening 213, wherein a wave height on one side of the rectangular corrugated ring 1032 is greater than a wave height on the other side and the wave height is gradually reduced, or an opening is formed on one side with a low wave height.

In this embodiment, referring to FIG. 25, the covered stent 100 further includes a transition section 1027 connected between the proximal section and the aneurysm sac covered section 102. When the proximal openings of the first embedded stent 21 and the second embedded stent 22 are configured as double oblique openings, the transition section 1027 is located between the proximal end of the aneurysm sac covered section 102 and the first proximal oblique opening 211 and the second proximal oblique opening 222. A transition stent 10271 is arranged on the transition section 1027, and the transition stent 10271 can support a covering membrane on the transition section 1027 formed between the double oblique openings, thereby avoiding a condition that the aneurysm sac covered section 102 lacks a support structure at this position to cause collapse or poor release.

In this embodiment, referring to FIG. 27 to FIG. 28, the transition stent 10271 is separately arranged on the transition section 1027, and a shape of the transition stent 10271 is matched with a shape of the transition section 1027. Herein, since a distal end, close to the support corrugated ring of the aneurysm sac covered section 102, of the proximal section 101 is a flat opening with a uniform wave height, while a V-shaped opening is formed between the first proximal oblique opening 211 and the second proximal oblique opening 222, and in order to ensure that the transition stent 10271 is flush with the proximal end of the aneurysm sac covered section 102 and the first proximal oblique opening 211 and the second proximal oblique opening 222 at a proximal end and a distal end, the proximal end of the transition stent 10271 includes a flat opening, while the distal end includes a convex V-shaped protrusion.

In some embodiments, the transition stent 10271 may be an annular stent, or may be a single sheet-like stent; when the transition stent 10271 is configured as the annular stent, at least two V-shaped protrusions are arranged symmetrically on two sides of the annular stent, and are configured to match the V-shaped opposite openings formed by the first proximal oblique opening 211 and the second proximal oblique opening 222, and the annular stent can provide improved overall support performance, not only provides support for the transition section 1027 but also can provide support for a junction between the proximal section 101 and the aneurysm sac covered section 102, such that the stability of the connection at a transition position between the proximal section 101 and the aneurysm sac covered section 102 of the covered stent 100 is enhanced; when the transition stent 10271 is configured as the single sheet-like stent, at least two sheet-like stents are symmetrically arranged on two sides of the V-shaped opposite openings formed by the first proximal oblique opening 211 and the second proximal oblique opening 222, and a proximal side of the single sheet-like stent is a flat side, and a distal side is a V-shaped side which has a convex middle and two gradually lowered sides; and the single sheet-like stents directly provide supporting force for the transition section 1027 from two sides, and can ensure that the transition section 1027 is supported and improved flexibility between the proximal section 101 and the aneurysm sac covered section 102 is achieved at the same time.

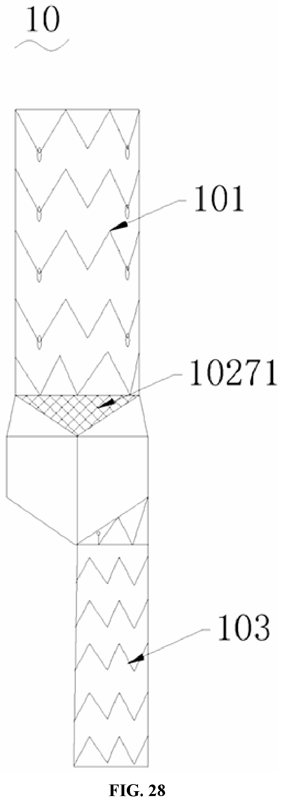

In one embodiment, referring to FIG. 27 and FIG. 28, the transition stent 10271 is a corrugated ring stent having a Z-shaped or W-shaped braided structure, and a structure of the corrugated ring stent is similar to a structure of a proximal support corrugated ring 1011 of the proximal section 101, such that the transition stent has a better connection with the proximal section 101 at this position, thereby improving the flexibility of the entire covered stent 100. In another embodiment, the transition stent 10271 is a mesh braided stent having a mesh braided structure, and the structure of the mesh braided stent is similar to structures of the first embedded stent 21 and the second embedded stent 22, such that the integrity of the aneurysm sac covered section 102 is higher; and in addition, support tension of the mesh braided stent is greater, such that an inner wall of the stent is smoother, to further ensure patency of blood flow at this position.

### Embodiment 8

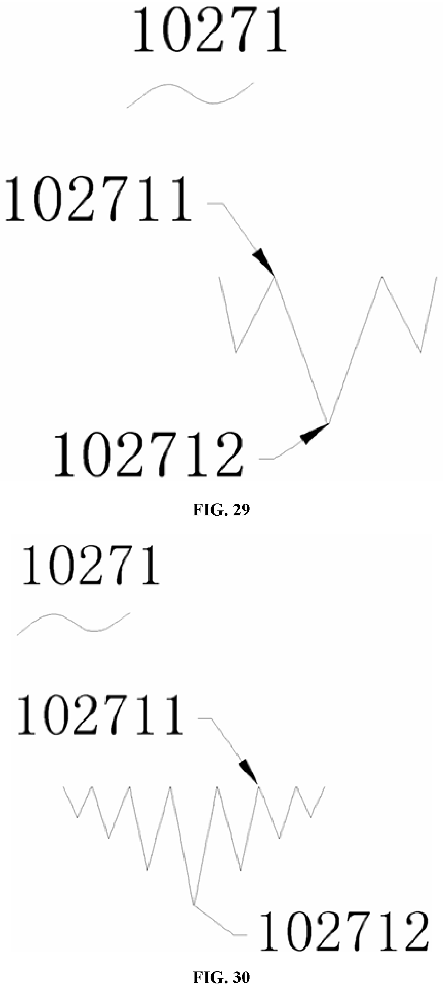

In this embodiment, referring to FIG. 29 and FIG. 30, structures of a main stent 10 and an embedded stent 20 are substantially the same as those in Embodiment 7, and a difference lies in that a transition stent 10271 is not separately arranged, a special-shaped corrugated ring 1012 is arranged at a distal end of a proximal section 101, and a portion of the special-shaped corrugated ring 1012 extends into an aneurysm sac covered section 102 and forms a support structure on a transition section 1027 to form the transition stent 10271. Specifically, a proximal end of the special-shaped corrugated ring 1012 has uniform proximal waves 102711 with equal heights, a distal end includes a plurality of distal high waves 102712 with unequal heights, the distal high waves 102712 protrude toward the distal end, and vertexes are flush with a first proximal oblique opening 211 and a second proximal oblique opening 222 of a first embedded stent 21 and a second embedded stent 22 to support the transition section 1027. At least two distal high waves 102712 of the special-shaped corrugated ring 1012 are arranged, the two distal high waves 102712 are symmetrically arranged on two sides of the distal end along a diameter of the special-shaped corrugated ring 1012, and the vertexes of the two distal high waves 102712 are close to bottoms of V-shaped opposite openings. Further referring to FIG. 29, a plurality of distal high waves 102712 of the special-shaped corrugated ring 1012 may be arranged, and mountain-like structures having a highest wave height in the middle and gradually lowered wave heights toward two sides are respectively formed on two opposite sides of the distal end of the special-shaped wave ring 1012, to match a shape of the transition section 1027. A support corrugated ring at the distal end of the proximal section 101 is configured as the special-shaped corrugated ring 1012, such that a condition that the transition section 1027 lacks a support structure to cause local collapse or poor release to affect patency of blood can be avoided, and a structure that the special-shaped corrugated ring 1012 extends between the proximal section 101 and the aneurysm sac covered section 102 enables the covered stent 100 to have more connection force between the proximal section 101 and the aneurysm sac covered section 102 and the integrity of the stent to be higher, thereby preventing bending at a transition position of the aneurysm sac covered section 102 and the proximal section 101.

### Embodiment 9

In this embodiment, structures of a main stent 10 and an embedded stent 20 are substantially the same as those in Embodiment 7 to Embodiment 8, and a difference lies in that a distal section 103 and an aneurysm sac covered section 102 of the main stent 10 are spliced in an adhesive bonding or suturing manner and then fixed, a first covering membrane 104 is arranged on a surface of the main stent 10, a second covering membrane 202 is arranged on a surface of the embedded stent 20, the first covering membrane 104 is a PET membrane, the second covering membrane 202 is an ePTFE membrane, and the distal section 103 and the aneurysm sac covered section 102 are configured in a splicing manner, and a surface covering membrane of the distal section 103 may also be an ePTFE membrane to further ensure the patency of an external iliac artery.

In one embodiment, a first embedded stent 21 and the distal section 103 are integrally formed; the distal section 103 directly extends into the aneurysm sac covered section 102 and is sutured to the aneurysm sac covered section 102; a portion, located in the aneurysm sac covered section 102, of a second channel 1022 forms the first embedded stent 21; and a portion located outside the aneurysm sac covered section 102 forms the distal section 103. In this way, a stent main body with the first embedded stent 21 and the distal section 103 integrally formed may adopt a corrugated ring stent or a mesh braided stent, and a surface covering membrane is an ePTFE membrane. Herein, integral forming means that the first embedded stent 21 and the distal section 103 are formed on the same ePTFE covering membrane, and the formed integral stent does not have a bonded structure or a sutured structure. If the first embedded stent 21 and the distal section 103 are integrally formed, when blood flow enters the second channel 1022 and flows toward an external iliac blood vessel, no obstruction or protrusion generated by a spliced structure is present in the channel to avoid affecting the smoothness of an inner wall of the channel, and further effectively improve the patency of the channel.

In this embodiment, referring to FIG. 26, proximal openings and/or distal openings of the first embedded stent 21 and the second embedded stent 22 are provided with developing members 203. The developing members 203 are arranged at the proximal openings and the distal openings of the first embedded stent 21 and the second embedded stent 22, and the arrangement of the developing members 203 can help an operator to quickly find a position of the aneurysm sac covered section 102, relative positions of the first channel 1021 and the second channel 1022, and a form change after compression through a developing device, and can also help the operator to quickly find an entry of an internal iliac stent, to achieve rapid and accurate implantation of the internal iliac stent.

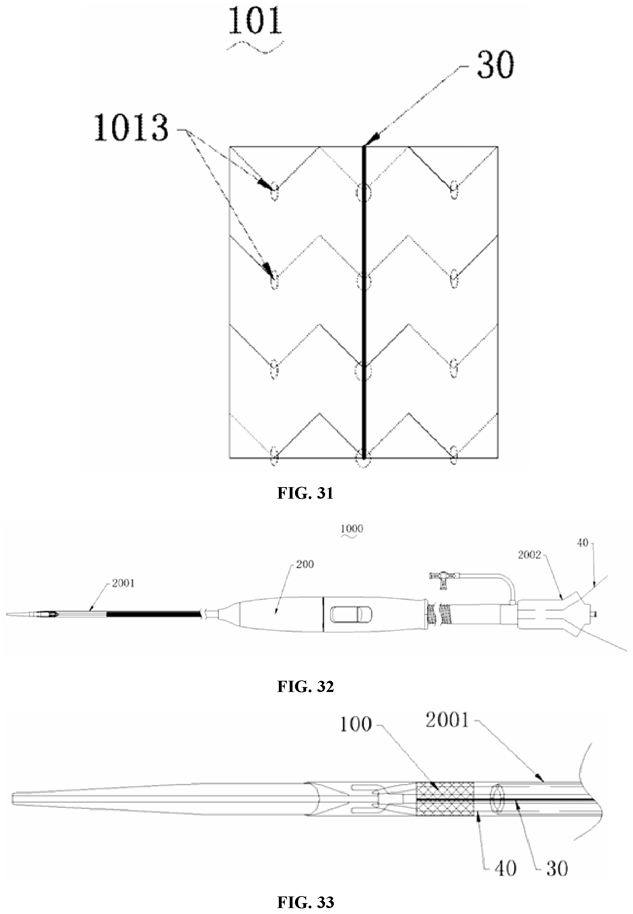

In this embodiment, referring to FIG. 31, in order to ensure that a release position can still be fine-adjusted when the position is inaccurate after initial release of the covered stent 100 provided by the present invention in a blood vessel, a plurality of hook members 1013 are arranged on a proximal section 101 of the main stent 10, and the plurality of hook members 1013 are arranged in an axial direction of the proximal section 101, and at least two hook members 1013 are arranged at the same axial position. In this way, the two hook members 1013 at the same axial position can be pulled to the same position and then hooked to a hook rod 30, and after the hook members 1013 at different axial positions are all hooked to the hook rod 30, at least the proximal section 101 of the covered stent 100 can be at least partially subjected to diameter-reduced constraint, such that the stent is in a partially compressed state and not completely released after initial release from a catheter of a delivery device. At this moment, if the release position is inaccurate, the release position can be fine-adjusted, and then the hook rod 30 is withdrawn to achieve complete release of the stent. The hook members 1013 may be annular hook members made of a polymer material, such as an annular wire made of a PET material; and the hook members may be fixed to proximal support corrugated rings 1011 of the proximal section 101 or a covering membrane in a suturing or adhesive bonding manner.

### Embodiment 10

Referring to FIG. 32 to FIG. 33, this embodiment provides a stent delivery system 1000. The stent delivery system 1000 includes the covered stent 100 as provided in Embodiment 1 to Embodiment 9, and further includes a delivery device 200. The delivery device 200 is configured to deliver the covered stent 100 of the present invention to a designated vascular location and release it, wherein the delivery device 200 typically includes a delivery sheath 2001 and a delivery handle 2002, and the delivery handle 2002 is configured to control the advancement and retraction of the delivery sheath 2001 to release the stent from the delivery sheath 2001. Referring to FIG. 31, the delivery sheath 2001 includes a hook rod 30, the hook rod 30 is configured for hook members 1013 on a proximal section 101 of the covered stent 100 to hook, and after hooking, at least the proximal section 101 of the covered stent 100 is in a partially compressed state.

In this embodiment, a pre-placed guide wire 40 is arranged in at least a first channel 1021 of an aneurysm sac covered section 102 of the covered stent 100, and the pre-placed guide wire is placed in the covered stent 100 in advance after the production of the covered stent 100 of the present invention is completed. In this way, after the stent is released, it is not necessary to reintroduce the guide wire. By arranging the pre-placed guide wire 40 in the first channel 1021 of the aneurysm sac covered section 102, guide wire insertion and guidance can be omitted, and an internal iliac stent can be quickly guided into a first embedded stent 21 of the first channel 1021 to be released directly through the guidance of the pre-placed guide wire 40, thereby improving the accuracy of release and shortening surgical time.

The above-described specific embodiments are only part of embodiments of the present invention, and are not limitations to the present invention; and the specification does not enumerate all embodiments with the concept of the present invention. Furthermore, part of features from different embodiments described above may be interchanged or combined. Those skilled in the art may also make simple substitutions based on actual requirements. The concept of the present invention is subject to the scope of protection as specified.

## Claims

1. A covered stent, comprising a main stent having a tubular body and an embedded stent, wherein the main stent comprises a proximal section, an aneurysm sac section, and a distal section in an axial direction; the proximal section is in communication with the distal section through the aneurysm sac section; the aneurysm sac section comprises a first channel and a second channel which are arranged in a radial direction, the second channel is in communication with the distal section, and a distal end of the first channel is provided with an opening in communication with the outside; the embedded stent is arranged in the first channel, the embedded stent is at least partially connected to a side wall of the first channel, and a distal end of the embedded stent is in communication with the opening; and the total support strength of the first channel and the embedded stent is lower than the support strength of the second channel.

2. The covered stent according to claim 1, wherein the aneurysm sac section comprises a plurality of first corrugated rings arranged at intervals in the axial direction, a number of waves of the plurality of first corrugated rings is the same, and crests and/or troughs of adjacent first corrugated rings are arranged opposite to each other.

3. The covered stent according to claim 2, wherein the first corrugated ring has an opening at least in a portion corresponding to the first channel.

4. The covered stent according to claim 2, wherein a wave angle of the first corrugated ring in a portion corresponding to the second channel is greater than a wave angle of the first corrugated ring in a portion corresponding to the first channel, or a wire diameter of the first corrugated ring in the portion corresponding to the second channel is larger than a wire diameter of the first corrugated ring in the portion corresponding to the first channel.

5. The covered stent according to claim 2, wherein the first corrugated ring comprises a plurality of first wave struts, and a support member is arranged between adjacent first wave struts, and a plurality of support members are configured such that a compressible distance between the first wave struts located in a portion corresponding to the second channel is smaller than a compressible distance between the first wave struts located in a portion corresponding to the first channel.

6. The covered stent according to claim 5, wherein a gap is formed between the support member and the adjacent first wave strut, and the gap in the portion corresponding to the second channel is smaller than the gap in the portion corresponding to the first channel.

7. The covered stent according to claim 5, wherein the support member is an elastic support member, two sides of the elastic support member are respectively connected to two adjacent first wave struts, and an elastic modulus of the elastic support member in the portion corresponding to the second channel is greater than an elastic modulus of the elastic support member in the portion corresponding to the first channel.

8. The covered stent according to claim 1, wherein the proximal section comprises a plurality of proximal support corrugated rings arranged at intervals in the axial direction, the distal section comprises a plurality of distal support corrugated rings arranged at intervals in the axial direction, and the embedded stent comprises a mesh main body.

9. The covered stent according to claim 1, wherein a first covering membrane covers a surface of the main stent, a second covering membrane covers a surface of the embedded stent, and the support strength of the first covering membrane is higher than the support strength of the second covering membrane.

10. The covered stent according to any one of claims 1 to 9, wherein the support strength of the aneurysm sac section located at a proximal end and a distal end is higher than the support strength of the aneurysm sac section at a middle position.

11. The covered stent according to any one of claims 1 to 9, wherein the support strength of the aneurysm sac section located at a proximal end or a distal end is higher than the support strength of the aneurysm sac section at a middle position.

12. The covered stent according to claim 1, wherein the embedded stent comprises a first embedded stent and a second embedded stent, the second channel is provided with the first embedded stent, the first channel is provided with the second embedded stent, and the first embedded stent is in communication with the distal section; a distal end of the aneurysm sac section is provided with an opening in communication with the outside, and a distal opening of the second embedded stent is in communication with the opening; and the support strength of the first embedded stent and the second channel is higher than the support strength of the second embedded stent and the first channel.

13. The covered stent according to claim 12, wherein the second channel accommodates the first embedded stent, the first channel accommodates the second embedded stent, the support strength of the first embedded stent is higher than the support strength of the second embedded stent, and/or the support strength of the second channel is higher than the support strength of the first channel.

14. The covered stent according to claim 13, wherein the first embedded stent and the second embedded stent each comprise a mesh main body, and the aneurysm sac section comprises a plurality of first corrugated rings arranged at intervals in the axial direction.

15. The covered stent according to claim 14, wherein a wire diameter of the first embedded stent is larger than a wire diameter of the second embedded stent, and/or a wire diameter of the first corrugated ring in a portion corresponding to the second channel is larger than a wire diameter of the first corrugated ring in a portion corresponding to the first channel.

16. The covered stent according to claim 14, wherein a grid density of the first embedded stent is greater than a grid density of the second embedded stent, and/or a wave angle of the first corrugated ring in a portion corresponding to the second channel is greater than a wave angle of the first corrugated ring in a portion corresponding to the first channel.

17. The covered stent according to claim 14, wherein an axial length of the second channel is greater than an axial length of the first channel, a distal end of the second embedded stent comprises an exposed section, and the exposed section passes through the opening.

18. The covered stent according to claim 17, wherein the first corrugated ring, at least in a portion corresponding to the same axial position as the exposed section, is provided with an opening in the second channel, and the opening faces the exposed section.

19. The covered stent according to claim 12, wherein a transition corrugated ring is arranged at a proximal end of the aneurysm sac section, and a proximal diameter of the transition corrugated ring is smaller than a distal diameter of the transition corrugated ring.

20. The covered stent according to claim 12, wherein a first covering membrane covers a surface of the main stent, second covering membranes cover surfaces of the first embedded stent and the second embedded stent, and the support strength of the first covering membrane is higher than the support strength of the second covering membrane.

21. The covered stent according to any one of claims 12 to 20, wherein the support strength of the distal section and the proximal section is higher than the total support strength of the aneurysm sac section, the first embedded stent, and the second embedded stent.

22. The covered stent according to any one of claims 12 to 20, wherein the support strength of the distal section or the proximal section is higher than the total support strength of the aneurysm sac section, the first embedded stent, and the second embedded stent.

23. The covered stent according to claim 1, wherein the aneurysm sac section comprises an aneurysm sac covered section, and the proximal section is in communication with the distal section through the aneurysm sac covered section; the proximal section and the distal section are provided with support corrugated rings; the embedded stent comprises a first embedded stent and a second embedded stent arranged in the radial direction in the aneurysm sac covered section, and the first embedded stent communicates the proximal section and the distal section; a distal end of the aneurysm sac covered section is provided with an opening in communication with the outside, and a distal opening of the second embedded stent is in communication with the opening; and the support strength of the first embedded stent is higher than the support strength of the second embedded stent.

24. The covered stent according to claim 23, wherein the first embedded stent and the second embedded stent each comprise a mesh main body, a wire diameter of the mesh main body of the first embedded stent is larger than a wire diameter of the mesh main body of the second embedded stent, and/or a grid density of the first embedded stent is greater than a grid density of the second embedded stent.

25. The covered stent according to claim 24, wherein at least an axial length of a portion, connected to the aneurysm sac covered section, of the first embedded stent is greater than or equal to an axial length of a portion, connected to the aneurysm sac covered section, of the second embedded stent.

26. The covered stent according to claim 25, wherein a proximal end of the first embedded stent is provided with a first proximal oblique opening, a proximal end of the second embedded stent is provided with a second proximal oblique opening, and the first proximal oblique opening and the second proximal oblique opening face each other.

27. The covered stent according to claim 26, wherein a distal end of the first embedded stent is provided with a first distal oblique opening which is parallel to the first proximal oblique opening, and a distal end of the second embedded stent is provided with a flat opening; or a distal end of the second embedded stent is provided with a second distal oblique opening which is parallel to the second proximal oblique opening.

28. The covered stent according to claim 26, further comprising a transition section connected between the proximal section and the aneurysm sac covered section, wherein a transition stent is arranged on the transition section.

29. The covered stent according to claim 28, wherein a proximal end of the transition stent is flush with a proximal end of the aneurysm sac covered section, and two sides of a distal end of the transition stent are respectively flush with the first proximal oblique opening and the second proximal oblique opening.

30. The covered stent according to claim 28, wherein a distal end of the proximal section comprises a special-shaped corrugated ring, a distal end of the special-shaped corrugated ring comprises a plurality of distal high waves, and the distal high waves extend to the transition section to form the transition stent.

31. The covered stent according to claim 23, wherein the first embedded stent and the distal section are integrally formed.

32. The covered stent according to claim 23, wherein proximal openings and/or distal openings of the first embedded stent and the second embedded stent are provided with developing members.

33. The covered stent according to any one of claims 1 to 32, wherein the proximal section is provided with a plurality of hook members, the hook members are arranged in an axial direction of the proximal section, and at least two hook members are arranged at the same axial position.

34. A stent delivery system, comprising the covered stent according to any one of claims 1 to 33 and a delivery device, wherein the delivery device comprises a hook rod, the hook members are connected to the hook rod, and after connection, the covered stent is maintained in at least a partially compressed state.
